Europäisches Patentamt

European Patent Office

Office européen des brevets

(11) Publication number: **0 107 111**
**A1**

# EUROPEAN PATENT APPLICATION

(21) Application number: 83109819.9

(22) Date of filing: 30.09.83

(51) Int. Cl.³: **A 01 K 5/00**

(30) Priority: 30.09.82 DK 4349/82

(43) Date of publication of application: 02.05.84
Bulletin 84/18

(84) Designated Contracting States: AT BE CH DE FR GB IT LI LU NL SE

(71) Applicant: **ADVANCED FEEDING SYSTEMS K/S, Vestergade 97-101, DK-5000 Odense (DK)**

(72) Inventor: **Blicher, Steen, Dalkildegard, DK-5600 Faborg (DK)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) **A method of feeding animals.**

(57) Animals, in particular pigs, are fed restrictively for a predetermined first period lasting at least one day so as to bring about increased cell division in the animal, which first period is followed by overfeeding the animal for another predetermined period, also lasting at least one day, so as to cause growth of the cells formed during the first period, and preferably alternating the periods of restrictive feeding and overfeeding until the animal has reached the desired weight, which occurs faster than when an animal is fed by a conventional feeding method.

To further the production of digestive enzymes, animals are subjected to sensory stimuli in the form of signals generated at each feeding place and varied in the course of a period of time so as to condition the animal to expect the supply of feed at a given moment. For pigs, the stimuli may comprise a reproduction of a sequence of the sow's suckling grunt accelerating towards the delivery of a mouthful of feed.

# A METHOD OF FEEDING ANIMALS

The present invention relates to a method of feeding animals, especially young animals.

From, e.g., British Patent Specification No. 2,056,837, it is known to increase the productivity in the production of animals, in particular pigs, by means of feeding the pigs at their respective feeding places during each feeding period with a portion of feed substantially corresponding to one mouthful or a few mouthfuls in such a way that the portions are supplied at such intervals that the amount of feed supplied corresponds to the desired eating rate of each animal, thus securing that each pig receives the same amount of feed.

Such a method of feeding ensures that all the pigs in the same sty are fed according to a desired standard, and that all pigs in the same sty have a relatively homogeneous growth development so that they reach the desired weight substantially simultaneously. The mouthful-wise dosage of feed influences the pigs to eat considerably more slowly than had been obtained previously in the known art. Also, by means of feeding pigs substantially mouthful-wise, competitive eating is prevented, thus obtaining an improved utilization of the feed ingested.

The feeding principle disclosed in the above-mentioned British Patent Specification No. 2,056,837, known as the "Biomat™" principle, can be said to mainly comprise a control of the number of feedings, and, during each feeding session, a control of the number of mouthfuls administered, and the lengths of the pauses between each mouthful to obtain a "fixation" of each pig at its feeding place. The beneficial effect of this, namely that each pig receives the same amount of feed, has been established to result in an increase of the feed utilization for the group of pigs by 7-9%. In particular for pigs which are older than the weaning age, the Biomat™ feeding principle has an additional important advantage in that the pigs respond to the feeding rhythm of the feeding apparatus by building up a conditioned reflex, by which the salivary glands and gastric glands start an increased production at the moment the pigs hear the start of the feeding apparatus. This reflex also builds up increased enzymatic and salivary production during the pauses between the mouthfuls. This conditioned reflex has been established to improve the feed utilization up to as much as additionally 15-20%.

The object of the method of the present invention is to increase the productivity in the production of animals even further than that obtained by the feeding method disclosed in the above-mentioned British patent. Thus, by the method of the present invention, it is possible to obtain a greater efficiency in the use of production facilities in that animals fed according to the method of the invention have a higher daily gain and therefore reach the desired weight faster than has been obtained in the known art.

In one aspect, the present invention relates to a method of controlled feeding of animals which comprises feeding each animal restrictively for a predetermined first period lasting at least one day, and preferably several days, so as to bring about increased cell division in the animal, which first period is followed by overfeeding the animal for another predetermined period, also lasting at least one day, so as to cause growth of the cells formed during the first period. The periods of restrictive feeding and overfeeding, respectively, may preferably be alternated until the animal has reached the desired weight.

In the present specification and claims, the term "controlled" is understood to mean that the periods of restrictive feeding and overfeeding are intentional and regulated. Preferably, the control of the feeding is performed on the basis of data or algorithms expressing relations between the average weight of the animals, the age of the animals, the previous administrations of feed to the animals, etc., in a manner which is known per se, for example from the above-mentioned British Patent No. 2,056,837. In the most practical embodiment, the algorithms and the data are coded into a computer which regulates the administration of the feed in accordance with the desired regimen.

In the present specification and claims, the term "restrictive feeding" or "feeding restrictively" is understood to mean that the amount of feed administered to the animal during the first period is lower than the optimal average previously determined for the particular animal species involved, and the race and age of the animal in question. The decrease in the amount of feed administered is normally in the range of 10-50%, such as 20-50%, especially 20-40%, such as 20-30%, calculated on the weight of the average optimal amount of feed.

Similarly, the term "overfeeding" is understood to mean that the amount of feed administered to the animal during the second period is higher than the optimal average previously determined for the animal in question, in particular 20-50% higher, especially about 30% higher.

The method of the invention utilizes the well-known principle of natural biorhythms in animals. One such biorhythm governs the way in which animals grow. Animal growth is not regular, but may substantially be divided into two phases: one phase during which division or multiplication of the body cells takes place without any actual growth of these cells, and another phase during which each of the cells obtained during the first period grows, and the growth continues until the cell has become sufficiently large to divide once more. In other words, upon cell division, each cell has a certain genetically determined growth potential.

It is believed to be possible to influence this natural biorhythm by the method of the present invention so that, if animals are fed restrictively, they will enter a phase where the major part of the available energy is utilized for cell division rather than cell growth and energy depositing. The increased cell multiplication activity may be of the order of about 20%, dependent on the reduction of the amount of feed administered to the animal. It is, however, important to keep in mind that this phase of cell division only occurs when the primary energy depots of the body have been used up. The length of the restrictive feeding period is thus relatively shorter with young animals, as these have only very small primary energy depots, if any, and proportionally longer with older animals. It is evident that the re-

4 $0107111$

strictive feeding should not be an inadequate feeding with such a low energy supply that the muscle tissue of the animals begins to degenerate.

When, after the period of restrictive feeding, the animal is administered an amount of feed which is larger than the optimal average determined for the particular animal, the growth potential of the cells formed during the first phase is utilized so that all the cells formed grow to normal size which causes a corresponding increase in the weight of the animal, which means that animals fed according to the method of the invention will eventually obtain an increase in weight which may be up to as much as e.g. 20% relative to animals which are, at all times, fed uniformly according to the determined optimal average.

In order to produce the desired effect it is advantageous to increase the amount of feed some time prior to the second phase in order to take into account the time lag before the increase of the amount of feed takes effect. (The factor determining the transition from the first period to the second period is, among other things, the increased amount of feed). The cycle of cell division and cell growth is closely connected with the hormonal level of the animal, i.e. the concentration of growth hormone from the pituitary gland, thyroxin from the thyroid gland, etc. It is advantageous that the feed intake in the overfeeding period is as high as possible. According to preferred embodiments of the invention, discussed in greater detail below, particular measures are available for stimulating the feed intake of the animals during the overfeeding period, such as controlled variation in the feed composition, or controlled use of stimuli.

As a general rule, the period of restrictive feeding should preferably extend over a period of days, such as 1-5 days, preferably 3-5 days, especially 3 days, and the period of overfeeding should last at least one day such as 1-5 days, and with respect to pigs, the period of overfeeding should typically last 2 days. However, depending upon the particular animal species and the particular conditions in question, suitable and optional periods may be ascertained by the person skilled in the art based on the present disclosure. Parameters which

5     0107111

may be employed to assess the effect of the feeding strategy and adjusting and optimizing the feeding schedule are, i.a., the following:

the weight of the animals, the daily weight gain of the animals, the consumed amount of feed units consumed per unit of weight gain, the character of the manure (in particular the consistency of the manure (avoidance of diarrhoea)), the amount of energy in the feed supplied, the amount of energy in excrement and urine, the amount of crude protein in the feed, the amount of crude protein in excrement and urine, and the percentage of pure meat in the animals ready for killing.

Although the pattern of restrictive feeding and overfeeding seems to be the most important factor influencing the above-mentioned natural biorhythm of the animal with respect to growth, it may also be possible to assist the shift in this biorhythm by means of exposing the animal to changes in external conditions and/or other factors influencing the animal in connection with alternating the feeding periods. In other words, the additional factors likely to influence the biorhythm of cell division and cell growth comprise factors which are principally directed to psychologically influencing the animal, such as changes in light, sound and heat stimuli, the smell, taste and consistency of the feed, the habitat of the animal and, to a minor degree, the composition of the feed.

The method according to the invention may be used for feeding animals which are placed in separate enclosures or which are placed in groups in the same enclosure. Thus, it may be advantageous to place social animals in groups as a sound and natural behaviour of a social animal is strongly dependent on the influences and interactions prevailing in the animal group (e.g., formation of hierarchy) and ultimately results in a higher yield of meat from each animal. If the animals are placed in groups it is important to ensure that each animal has access to a feeding place without being displaced by the stronger animals in the group so that each animal receives the stipulated amount of feed. The homogenous consumption of feed may be ensured by using the feeding principle disclosed in the above-mentioned British patent. The method according to the present invention may, however, also be used in connection with any conventional feeding method.

Thus, in a particularly advantageous embodiment, feeding sessions according to the restrictive feeding/overfeeding strategy of the present invention are performed by simultaneously feeding a plurality of untethered animals at their respective feeding places in the same enclosure in which all feeding places are freely accessible to all the animals, and supplying portions of feed to each of the feeding places during a feeding period at intervals ensuring that the amount of feed supplied to each feeding place substantially corresponds to a desired predetermined eating rate of the animals. Placing untethered animals in groups in the same enclosure has the added advantage that the animals develop a synchronized biorhythm so that by restrictive feeding and overfeeding of the animals, respectively, a synchronized feed utilization is obtained.

The method according to the present invention is especially advantageous when used to feed young animals, that is, pre-pubescent animals, e.g., mammals at the weaning stage, or poultry such as chickens or turkeys, as young animals have an optimal growth potential. Thus, an optimal feeding according to the principle of the invention ensures an optimal utilization of the feed administered to the animal. In young animals, even large amounts of feed ingested are more likely to convert to meat rather than fat than is the case with older animals, thus improving the slaughtering quality of the animal.

It is generally known that animals learn to respond to a given signal very quickly if they receive a reward. This learning capacity of the animals may be utilized to positively influence the behaviour pattern, especially the eating reflex, of the animals. Such influence is mainly connected with stimulating the sensory apparatus of the animals so that the specific stimuli given may affect the visual, auditory, tactile, gustatory or olfactory sense. As indicated above, the advantage of stimulating the animals' eating reflex is due to the fact that such a sensory stimulation enhances the production of digestive enzymes thus ensuring an optimal utilization of each mouthful of feed administered.

0107111

For instance, it is well-known that pigs are very observant to different sounds, and in accordance with what has been stated above, if,
e.g., the food dispensing valve of the feeding place opens with a
click before feeding, and if, furthermore, this valve opens before the
distribution of each portion of feed, it has been noted that each click
increases the pigs' interest in the feeding trough, as they have
learned that the click is shortly followed by a portion of feed.

According to another aspect of the invention, the utilization of stimuli
is intensified to obtain an improved feed utilization and a better daily
gain based on an improved utilization of the conditioned reflexes of
the animals. In this aspect, the invention relates to a method of
feeding a plurality of untethered animals at their respective feeding
places in the same enclosure where all feeding places are freely
accessible to all the animals, the method comprising supplying portions of feed to each feeding place during a feeding period at intervals ensuring that the amount of feed supplied to each feeding place
substantially corresponds to a desired predetermined eating rate of
the animals, and generating a signal at each feeding place over a
period of time preceding the supply of each portion of feed, the
signal being varied in the course of this period of time so as to
condition the animal to expect the supply of each portion of feed at a
given moment.

The signal may be an acoustic signal, i.e. a signal which affects the
auditory sense of the animals, but it may also be a signal which
affects the visual, tactile, gustatory or olfactory sense of the animal,
or any combination of these influences may be utilized. The variation
of the signal may be a variation in intensity or frequency of the
signal, and/or it may be a superimposition of a signal on other types
of signal. In practice, visual and auditory signals will normally be
most suitable for the purpose, although it cannot be precluded that
for certain animals, tactile signals may be employed to advantage.

In this aspect of the present invention, which may to be employed to
advantage in connection with the restrictive feeding/overfeeding
method disclosed above, *inter alia* because it enhances the animals'

8

0107111

acceptance and utilization of the overfeeding, but which is not limited to being employed in connection with the restrictive feeding/overfeeding method, the principle of stimulating the animal's sensory apparatus has been developed further in order to increase the patience of the animal to be fed in that, according to the present invention, the signal may be extended over a period of time and varied in the course of this period whereby the animal is made to await the moment when the portion of feed is supplied to the feeding place and conditioned to receive and utilize the portion of feed. Thus, by means of stimulating the animal's interest, it may be possible to introduce a prolonged pause between each portion to secure a longer period of feed consumption since, when for instance a prolonged and varied acoustic signal is generated, the animal may be fixated at its feeding place for a relatively longer period in expectation of a reward, i.e. the next portion of feed. Without such a signal, the interval between portions would necessarily have to be relatively brief as the animal would become impatient and begin to change places with the other animals in the enclosure. Thus, the positive effect of stimulating the production of digestive enzymes would be lost.

The feed which is administered to the animals in accordance with either or both of the above-disclosed methods may be any feed composition suitable for the animals in question at the particular stage of development in question. However, when the methods of the invention are utilized for feeding young animals, an additional beneficial effect with respect to the development and weight gain of the animals is obtained by adapting the composition of the feed to the development of the animals in accordance with the principles disclosed in International Patent Application No. PCT/DK82/00023 and equivalent national applications, all of which claim priority from Danish Patent Application No. 1194/81.

In accordance with these principles, the ratio between digestible protein and other feed constituents, in particular carbohydrates, in the feed of the animals is adapted to the development of the animals, through at least substantially daily adjustment of the said ratio. As appears from the following, it is preferred that the ratio be adjusted substantially at each feeding.

P&V F3104 jB int. tekst KBM/OP/KPJ/P 1983 09 29/7

0107111

In one main aspect, this principle is characterized in that a mixture comprising digestible protein is fed in fluid form and a basic feed comprising carbohydrates is fed as a solid material, and that the ratio between digestible protein and other constituents, in particular carbohydrate, in the total feed consisting substantially of the combination of the fluid mixture and the basic feed is adjusted to the development of the digestion system of the animals through at least substantially daily adjustment.

According to this principle, the adjustment of the ratio between digestible protein and carbohydrate is performed in a most suitable and convenient manner by adjustment of the ratio between the said fluid mixture and the said basic feed.

In the following, reference will be made only to pigs, in particular pigs of up to 40 kg or even porkers up to slaughtering weight, as the animals to be restrictively fed/overfed and/or influenced by sensory stimuli and optionally to be fed according to the above-mentioned principle of being fed with a mixture comprising digestible protein in fluid form and a basic feed comprising carbohydrates as a solid material and, optionally, adjusting the ratio between digestible protein and other constituents through at least substantially daily adjustment, although it will be understood that other species of animal may also be fed according to the methods of the invention.

During experiments with the learning capacity of pigs, it has been observed that an acoustic signal such as a click is often not sufficient to hold the attention of very young pigs. According to a preferred embodiment of the present invention, the signal which is varied in intensity prior to the administration of a portion of feed may be a reproduction of a series of sow suckling grunts with the grunt frequency increasing towards the administration of a portion of feed. Between the portions, it is also preferred to reproduce a series of sow suckling grunts with increasing grunt frequency.

A typical reproduction of a sow suckling grunt series for use in the method of the invention is as follows: About 1-2 minutes prior to the

P&V F3104 jB Int. tekst KBM/OP/KPJ/P 1983 09 29/7

administration of the first feed portion, grunts are emitted at a frequency of about one grunt per 2.5 seconds. About 10-25 seconds prior to the administration of the first portion, the grunting rate is accelerated so that the grunt frequency is about one grunt per 0.6 seconds immediately prior to the administration of the feed portion, the grunting being stopped exactly at the moment when the feed portion is administered. Then, there is a pause of a duration of a few seconds, e.g., about 5 seconds, and then, a grunting sequence corresponding to the last seconds, e.g., the last 5 seconds, of accelerating grunts prior to the administration of the first feed portion is re-emitted; this procedure is repeated prior to each portion of feed.

The sow suckling grund is a characteristic and very specific grunting sound produced by the sow when suckling her pigs. A detailed description of the sow suckling grunt and grund sequence is given in "Confinement and continuous noise as environmental factors affecting communication in the domestic pig", by Per Jensen, published by the Swedish University of Agricultural Sciences, Faculty of Veterinary Medicine, Department of Animal Hygiene, Report 8, Skara, 1983.

The sow's suckling grunt may for example be recorded and broadcasted in the pig-sty by means of reproduction equipment placed in the feeding machines. The reproduction equipment may, e.g., comprise a mechanical, magnetic or semiconductor storage in which the grunt frequency is stored. It is also possible to install reproduction equipment generating other acoustic signals in a similar way so as to provide the signal most optimal to the particular stage of development of the pigs. Also, the frequency and intensity of the acoustic signal may be adjusted to the optimal influencing level. For example, the sound may be produced with increasingly shorter intervals until the moment of feed supply in order to raise the pigs' expectation of a reward which, as mentioned above, furthers the production of digestive enzymes.

However, the feeding frequency may not be uniform throughout the pigpen. Pigs which have just been taken from the sow are fed more frequently than pigs which are somewhat older. It is normally not possible to differentiate between the feeding frequencies of the individual sties in the same pigpen by means of acoustic signals as such a signal will normally be audible throughout the pen and, consequently, will cause agitation among the pigs which are not fed when the signal is generated. If, on the other hand, the pigs are taught to respond to a combination of light and acoustic signals instead so as to expect to be fed only when both the acoustic signal and the light signal is generated, which may be done individually in each sty, any additional acoustic signals will eventually be ignored by the pigs.

Thus, according to the principle of the invention, it is possible to stimulate the pigs' interest in the feeding place by means of light signals as well as acoustic signals so as to supplement the acoustic signals provided in the interval between each mouthful with light signals or to substitute light signals for acoustic signals altogether. A gradually increasing intensity of light towards the moment when the next mouthful is provided is likely to result in a similar effect as described above with respect to fixation and conditioning of the pigs as when an acoustic signal is produced. Thus, the provision of a light signal will produce the same effect of stimulating the production of digestive enzymes, thus optimizing the digestive process.

It is known that young pigs are attracted by thermal radiation. When pigs are born, they are directed to the sow's belly by the increased heat radiation from her belly relative to the heat radiation from her fat-insulated back. If the pigs are weaned very early (at about 2-3 weeks) if may be envisaged that the pigs will be drawn to the feeding place if the above-mentioned signals are combined with thermal radiation from the feeding machine. The thermal radiation may advantageously be combined with the light signals, e.g. by installing a sunlamp-like apparatus in the feeding machine.

By applying the feeding method described above, in particular with respect to the variations in sensory stimuli, it is possible to introduce longer feeding periods as the animals no longer engage in competitive eating since their interest becomes directed solely to their respective feeding places, and the interval between each mouthful may be prolonged to ensure an optimal utilization of the feed consumed. Thus, in contradistinction to the known feeding methods where pigs are fed about 3 times a day for a period of about 3-5 minutes, it is now possible to feed pigs according to the principles of the present invention for any period of time per feeding session. For example, it is possible to feed pigs of a weight of 25-90 kg for periods of up to, e.g. 20 minutes at each feeding session, with at least 6 feeding sessions a day, thereby obtaining a better feed conversion and a higher daily gain. On the other hand, it is also possible to feed weaning pigs 6-18 times a day for correspondingly shorter feeding sessions, such as feeding sessions of a duration of about 2.5 minutes 18 times a day, which is then gradually changed into about 20 minutes 6 times a day when the pigs have a weight of about 20-30 kg.

Particularly during the weaning period, the digestive system of the pigs is subject to a quick and dramatic development with respect to the capacity to produce digestive enzymes. As an example, the intestine of a weaning pig grows about 20 cm per day. Known feeding methods are not sufficiently adjusted to this quick change. According to a preferred principle of the present invention, the pigs, in particular during the weaning period, are fed with a feed mixture adjusted to the enzyme production of the pigs or to a desired development of the enzyme production of the pigs by frequent adjustments of the composition of the feed, the quantitative administration of the feed and the intervals at which the feed is administered. As indicated above, it is preferred to adjust the composition of the feed at least daily during the weaning period, and indeed, in a preferred embodiment, an adjustment of the composition is performed during the individual feedings.

As mentioned above, the adjustment of the composition of the feed may easily be obtained by mixing the feed for each feeding from at least three stock constituents, one of which being a normal carbohydrate-rich feed such as gritting, the other one being a fluid mixture having a high content of digestible protein or available protein, in particular a high content of protein of animal origin, and the third one being water, optionally containing additives.

P&V E3104 iB int. tekst KBM/OP/KPJ/P 1983 09 29/7

Thus, in this principle of the invention, the total consumption of the pigs may be a mixture of e.g. water, a carbohydrate-rich conventional feedstuff such as gritting and a fluid feed material with a high content of digestible protein, in particular protein from animal sources. The adjustment of the composition of the feed and/or the quantitative administration of the feed for the development of the digestion system of the pigs or for a desired accelerated development of the digestive system of the pigs is preferably performed through at least daily adjustments of the total composition fed to the pigs during a feeding session ("macroadjustment"), and, according to the above-mentioned preferred embodiment, through adjustment of the ratio between available protein in fluid form and other constituents, in particular carbohydrates, even during the individual feeding ("microadjustment"). The macroadjustment may be performed in strict accordance with a curve which represents the optimal ratio between digestible protein and other feed constituents, in particular the ratio between digestible protein and carbohydrate. This optimum curve may be the curve which is known or may be found to be true for the animals in question under normal, favourable development, or it may be a curve which represents the optimal conditions during a development which is deliberately accelerated using the principle of the present invention. In both cases, it is important to perform the macroadjustment of the feed for the desired development of the pigs very frequently and during the weaning period preferably at least daily and most preferably substantially at each feeding, as a less frequent adjustment would mean that the pigs receive a feed which is not optimal for their development and/or that parts of the feed composition are in fact wasted; a less frequent adjustment would also mean more dramatic changes in the food composition at each adjustment which may cause stomach upset.

In connection with the restrictive feeding/overfeeding strategy in particular, it is envisaged to be advantageous to adjust the ratio between carbohydrate and protein constituents in the feed in such a manner that the carbohydrate-rich constituent is admixed with a lower amount of protein (typically an average reduction of 10-15% relative to the standard intake of protein for the stage of development in question)

0107111

during the restrictive feeding period, while the amount of protein is increased during the overfeeding period, typically an average increase of 25-30% on the same basis.

As mentioned above, the feed composition may also advantageously be changed during the individual feeding (microadjustment) so that in addition to the establishment of the optimum feed composition adapted to the development stage of the animal as secured through the ratio between the total amounts of the feed constituents delivered during a feeding, a dynamic adjustment of the feed composition to the relative capacity (relative with respect to the feed composition) of the digestion system is obtained during the feeding. Such adjustment during the feeding will normally be one which delivers a relatively higher content of digestible protein and a relatively lower content of carbohydrates in the feed at the beginning of the feeding and a relatively lower content of digestible protein and a relatively higher content of carbohydrate at the end of the feeding.

In a preferred embodiment of the method according to this principle, the feed is mixed from stock constituents of the kind mentioned above individually from pigsty to pigsty and from day to day, preferably from feeding to feeding, (and, according to one preferred embodiment, the composition is "microadjusted" during the individual feeding) by means of a feeding apparatus which provides mouthful-wise dosage, preferably a feeding apparatus of the type described in the above-mentioned British Patent No. 2,056,837 or any other apparatus capable of performing the method described in the above-mentioned patent applications.

The ingredients employed may e.g. be water, gritting and a fluid supplemental feed material with a high content of digestible protein. A suitable composition of the fluid supplemental feed material results in the attainment of the above optimization of the foodstuffs with respect to the ability of the pigs to utilize them when the ratio between the supplemental feed material and the gritting is varied.

0107111

Also, the fluid form of the protein-rich supplemental feed material makes it possible to add, in advance, enzymes such as pepsin which will function partly as a pre-digestion and partly as an accelerator of the development of the digestion system of the pigs.

In addition, additives of various conventional kinds such as trace minerals, iron supplements, growth promoting antibiotics, and/or therapeutic drugs or microorganism cultures may be added to the fluid supplemental feed material.

According to one aspect of this principle, the fluid feed material used as a protein-rich supplemental feed substantially consists of constituents which have not been exposed to any substantial drying treatment. Such a feeding material may easily be administered by means of, e.g., a feeding apparatus of the type disclosed in the above-mentioned British patent and may - when the ratio between administration of liquid and solid feed is adjusted in the feeding apparatus - be used as one stock constituent for the above-mentioned frequent adjustment of the total feed composition for optimum adjustment to the development of the pigs.

An important example of a fluid protein-rich supplemental feed material is a "protein soup" which is mainly based on such materials as blood, slaughterhouse waste, partially digested intestine content from slaughterhouses, industrial protein waste, e.g. from the canned food industry or the snack industry, and household food waste, preferably nutritively balanced with such protein-rich constituents as fish pulp, whey, yeast cream, skim milk, etc. Such a fluid supplemental feed material may, e.g., utilize the very considerable amounts of waste products from the slaughterhouses and dairies without the expensive and often partly destructive working up process, and the material may wholly or partially replace expensive feed materials such as dried protein.

In the methods of the invention, the feeding rhythm and feeding mechanism is preferably adjusted to be in the best possible accordance with the development of the pigs. Thus, the weaning period may start by feeding with same frequency as sows suckle the pigs, i.e. frequent at the beginning and then little by little and gradually increasing the time intervals between the feedings.

Also, the feeding rhythm may, according to one embodiment of the invention, be changed during each individual feeding by changing the interval between the mouthfuls, in order to obtain a dynamic adaption of the rate at which the mouthfuls are served to the capacity of the pigs during each feeding session.

The fluid supplemental feed material according to the above-mentioned principle is preferably composed in such a manner that solely by being combined with the carbohydrate-rich constituent, e.g. barley gritting, - and with the water dosed at the feeding - in a varying ratio, it may meet the nutritive demand for every stage of development for pigs from 2 weeks or 3 weeks and up till slaughter weight. Thus, one single supplemental feed material meets the total demand for supplemental feed during the whole breeding period. Another possibility constituting a preferred embodiment is to work with a special supplemental feed material which nutritively is composed so that it meets the above-mentioned demands during the weaning period, (that is, normally during the period where the pigs are from 2 to 12 weeks old, but also including the cases where the pigs are weaned in a very young age such as the second day after farrowing).

The fluid supplemental feed material of the invention may as previously mentioned be based, e.g., on blood from slaughterhouses and slaughterhouse waste which has been sufficiently comminuted and distributed in the blood. Also dairy waste, e.g. whey and casein waste from cheese production, is a good basis for the supplemental feed material. Raw fish pulp may suitably also be a constituent of the fluid supplemental feed material, in that, in addition to showing the well-known valuable protein composition, it also has a beneficial influence in that it contributes to lower the viscosity of the supplemental feed, and in that the pepsin from the fish mass has a hydrolyzing effect on the proteins. Household food waste, suitably obtained from households where the waste is sorted in a food waste part and a non-food waste part, constitutes another interesting source of available protein, and will often show an amino acid balance which may be utilized to balance the final amino acid balance of the fluid supplemental feed material.

The production of the supplemental feed material will usually simply consist in a mixing of the constituents, if necessary with communition of possible solid constituents which are to be distributed in the resulting fluid material. The resulting "protein soup", which normally has a dry matter content of about 20-45%, typically about 25-40%, is thereafter normally autoclaved. If the protein soup has a too high viscosity at room temperature, enzymes may be added to lower the viscosity. Preservation of the protein soup may be performed by adjusting the pH to an acidic value in a manner known *per se* by addition of an acceptable acid such as formic acid. Another preservation method is addition of microorganism cultures which will generate acid to lower the pH, e.g., lactic acid-producing bacteria. Examples of bacteria which are suitable for this purpose are *Lactobacillus acidophilus*, *Lactobacillus plantaro*, *Lactobacillus lactis*, or *Streptococcus lactis* or *Streptococcus cremoris*. The preservation of the protein soup may also be obtained by autoclaving or by using normal preserving additives.

The fluid supplemental feed material may suitably be made by the same known processes as are used for preparing dried protein-rich animal feed from the same types of materials, but omitting the final drying stage.

In order to obtain a standardization of the fluid supplemental feed material and to avoid that variations in the delivery of raw materials will result in too great variations in the final fluid material, it may be desirable to divide the fluid or the raw materials into components according to composition, e.g., into fat, protein, and collagen water, etc., and thereafter to combine these components in desired ratios to obtain a standardized product.

The character of the supplemental feed and the way in which it is prepared make it possible to add desired additives, such as enzymes, microorganisms, vitamins, which would be completely or partially destroyed in the conventional processes for manufacturing pig feed.

In many cases, it may be advantageous to start the feeding of pigs at the beginning of the weaning period with protein soup only and then, as soon as the pigs have been succesfully started, to "train" their enzyme system (induce the production of enzymes decomposing vegetable matter) by administering a carbohydrate-rich feed in increasing proportions. Gritting has been found to be a suitable carbohydrate source for this "enzyme training".

Once the pigs have been successfully started using a high proportion of digestible protein, their development will be faster and they will sooner become able to utilize carbohydrate and non-animal protein.

When a "microadjustment" is performed, this may typically comprise addition of proportionally more protein soup at the start of the feeding (e.g., about 50-75% more than the nominal amount to be the average), and proportionally less during the later minutes of the feeding, so as to obtain the average desired according to the "macroadjustment". In this manner, the capacity and secretion of the digestion system of the pigs is optimally utilized.

In addition to the above-mentioned parameters improving the feed utilization and weight gain of the pigs, it may also be advantageous, as an additional parameter, to institute a diurnal rhythm in which a considerable proportion of each day's feed ration, particularly about 50%, is administered early in the day such as between 6 a.m. and 12 a.m., and the remainder of the feed is administered at regular intervals throughout the rest of the day. This may preferably be accomplished in practice by coding the desired cycle and diurnal rhythm into a computer which controls the feeding system.

As an example, a combination of one kg of basic feed and one kg of protein soup (which may e.g. be used for pigs having a weight of about 7-9 kg) contains the following per feed unit:

|                        |          |       |
|------------------------|----------|-------|
| digestible fat         | 59       | grams |
| digestible protein     | 176      | grams |
| digestible lysine      | 10.4     | grams |
| digestible methionine  | 3.5      | grams |
| digestible cystine     | 2.3      | grams |
| calcium                | 11.5     | grams |
| phosphorus             | 9.3      | grams |

For pigs of an age of 10-11 weeks, weight about 20-24 kg, a preferred combination (1 kg of basic feed and 0.5 kg of protein soup) contains the following constituents per feed unit:

|                        |       |       |
|------------------------|-------|-------|
| digestible fat         | 39    | grams |
| digestible protein     | 160   | grams |
| digestible lysine      | 8.9   | grams |
| digestible methionine  | 3.0   | grams |
| digestible cystine     | 2.4   | grams |
| calcium                | 7.3   | grams |
| phosphorus             | 7.0   | grams |

(In the present specification and claims, the term "feed unit" indicates a Scandinavian feed unit which is equivalent to the energy content of "1 kg of barley", more precisely stated 7730 kJ (or 1845 kcal).

Examples of suitable protein soups appear from the following prescriptions:

0107111

## PRESCRIPTION 1

A protein soup was made from the ingredients stated in Table 1 in the ratios stated in Table 1. The ingredients were mixed in a vessel with disintegration until a fluid of suitable viscosity had been obtained. The fluid was thereafter sterilized by autoclaving.

### Table 1
#### Composition of Protein Soup

|  | per cent | kg dry matter | feed units | g Ca | g P |
|---|---|---|---|---|---|
| Blood | 30.0 | 5.7 | 7.5 | - | - |
| Slaughter waste | 50.0 | 15.0 | 12.5 | 1500 | 750 |
| Technical fat | 8.0 | 8.0 | 23.6 | - | - |
| Fishmeal | 5.0 | 4.7 | 7.1 | 175 | 111 |
| Skim milk powder | 5.0 | 4.8 | 6.5 | 68 | 49 |
| Additives *) | 2.0 | - | - | - | 190 |
| per 100 kg | (100.0) | 38.2 | 57.2 | 1743 | 1100 |
| per kg |  | 0.38 | 0.57 | 17.4 | 11.0 |
| per feed unit |  | 0.67 | 1.00 | 30.5 | 19.2 |

*) 1.0% monosodiumphosphate, 0.1% methionine, 0.5% vitamin/trace mineral mixture from Løvens Kemiske Fabrik, Denmark, 0.4% NaCl

The content of fat, digestible protein and the amino acids lysine, methionine and cystine in the protein soup derived from each of the constituents of the soup appear from Table 2.

0107111

## Table 2
### Content of Digestible Components in Protein Soup

| | kg dry matter | gramme digestible | | | | |
| --- | --- | --- | --- | --- | --- | --- |
| | | fat | protein | lysine | methio-nine | cystine |
| Blood | 5.7 | 90 | 4050 | 372 | 30 | 36 |
| Slaughter waste | 15.0 | 1100 | 5000 | 230 | 55 | 25 |
| Technical fat | 8.0 | 7040 | - | - | - | - |
| Fish meal | 4.7 | 356 | 3300 | 261 | 92 | |
| Skim milk powder | 4.8 | 45 | 1685 | 131 | 40 | 13 |
| Additives *) | - | - | - | - | 100 | - |
| per 100 kg | 38.2 | 8631 | 14035 | 994 | 317 | 107 |
| per kg | 0.38 | 86 | 140 | 9.9 | 3.2 | 1.1 |
| per feed unit | 0.67 | 151 | 245 | 17.4 | 5.5 | 1.9 |

*) 1.0% monosodiumphosphate

0.1% methionine

0.5% vitamin/trace mineral mixture, from Løvens Kemiske Fabrik, Copenhagen, Denmark

0.4% NaCl

A basic feed mixture utilizing this protein soup may be made from barley meal and soy meal in the ratios stated in Table 3. Table 3 also contains the corresponding values for feed units and the content of calcium and phosphorus derived from the barley and the soy meal, respectively.

0107111

Table 3

Composition of Basic Feed

|  | per cent | feed units | g Ca | g P |
|---|---|---|---|---|
| Barley | 82 | 82.0 | 48 | 261 |
| Soy meal | 18 | 20.5 | 52 | 117 |
| per 100 kg | (100) | 102.5 | 100 | 378 |
| per kg |  | 1.03 | 1.0 | 3.8 |
| per feed unit |  | 1.00 | 1.0 | 3.7 |

The content of digestible fat, digestible protein, and the amino acids lysine, methionine and cystine in the the barley and soy meal appears from Table 4.

Table 4

Content of Digestible Components in Basic Feed

|  | gramme digestible | | | | |
|---|---|---|---|---|---|
|  | fat | protein | lysine | methionine | cystine |
| Barley | 834 | 6970 | 246 | 123 | 148 |
| Soy meal | 86 | 7200 | 430 | 115 | 113 |
| per 100 kg | 920 | 14170 | 676 | 238 | 261 |
| per kg | 9.2 | 142 | 6.8 | 2.4 | 2.6 |
| per feed unit | 9.0 | 138 | 6.6 | 2.3 | 2.5 |

0107111

The protein soup of Tables 1 and 2 and the basic feed of Tables 3 and 4 may be combined in varying amounts and used for feeding of weaning pigs.


PRESCRIPTION 2

A protein soup may be made from the ingredients stated in Table 5 in the ratios stated in Table 5. The ingredients are mixed in a vessel with disintegration until a fluid of suitable viscosity had been obtained. The fluid is thereafter sterilized by autoclaving.

### Table 5
### Composition of Protein Soup

| | per cent | kg dry matter | feed units | g Ca | g P |
|---|---|---|---|---|---|
| Blood | 15.0 | 2.9 | 3.8 | - | - |
| Slaughter waste | 39.7 | 11.9 | 9.9 | 1191 | 596 |
| Fish pulp | 20.0 | 6.2 | 12.0 | 80 | 100 |
| Skim milk | 20.0 | 1.8 | 2.4 | 30 | 20 |
| Technical fat | 3.0 | 3.0 | 8.9 | - | - |
| Monosod.phosph. | 1.0 | 1.0 | - | - | 190 |
| Methion.mixt.10% | 1.0 | 0.9 | - | - | - |
| Carbavit Mikro 4001* | 0.3 | 0.3 | - | - | - |
| per 100 kg | (100.0) | 28.0 | 37.0 | 1301 | 906 |
| per kg | | 0.28 | 0.37 | 13.0 | 9.1 |
| per kg dry matter | | 1.00 | 1.32 | 46.5 | 32.4 |
| per feed unit | | 0.76 | 1.00 | 35.2 | 24.5 |

\* Wheat bran admixed with vitamins, antioxidants, trace minerals, and taste-improving agents.


P&V F3104 JB int. tekst KBM/OP/KPJ/P 1983 09 29/7

The content of fat, digestible protein and the amino acids lysine, methionine and cystine in the protein soup derived from each of the constituents of the soup appear from Table 6.

### Table 6
### Content of Digestible Components in Protein Soup

| | kg protein | gramme digestible | | | | |
| --- | --- | --- | --- | --- | --- | --- |
| | | fat | protein | lysine | methio-nine | cystine |
| Blood | 2.3 | 45 | 2025 | 186 | 15 | 18 |
| Slaughter waste | 6.0 | 873 | 3970 | 183 | 44 | 20 |
| Fish pulp | 3.1 | 2200 | 2720 | 210 | 74 | 30 |
| Skim milk | 0.7 | 20 | 660 | 54 | 12 | 6 |
| Technical fat | - | 2640 | - | - | - | - |
| Methion.mixt 10% | - | - | - | - | 100 | - |
| per 100 kg | 12.1 | 5778 | 9375 | 633 | 245 | 74 |
| per kg | 0.12 | 58 | 94 | 6.3 | 2.5 | 0.7 |
| per kg dry matter | 0.43 | 206 | 335 | 22.6 | 8.8 | 2.6 |
| per feed unit | 0.33 | 156 | 253 | 17.1 | 6.6 | 2.0 |

This protein soup may be used in the same manner as the protein soup according to Prescription 1.

0107111

PRESCRIPTION 3

In a similar manner as described above, a preferred protein soup is prepared from animal waste products and additives of minerals and vitamins. The protein soup has a dry matter content of about 30% by weight and has the following composition per feed unit:

280 g protein (digestibility about 90%)

As minimum values:

10 g digestible lysine

6 g digestible methionine*

6 g digestible cystine*

6 g digestible threonine

40 g fat

12 g Ca

10 g P

4 g NaCl

160 mg Fe

100 mg Zn

50 mg Mn

100 mg Cu

0,2 mg I

0,1 mg Se

4000 I.U. (International units) Vitamin A

400 I.U. Vitamin D

30 mg Vitamin E

2 mg Thiamine

5 mg Riboflavin

4 mg Pyridoxine

40 mg Niacin

15 mg Pantotenic acid

20 µg Vitamin $B_{12}$

(If the soup is not made from constituents (such as slaughterhouse waste) containing digestive enzyme, a mixture of such enzymes may be added as such).

* Methionine and cystine may partially replace each other.

P&V F3104 jB int. tekst KBM/OP/KPJ/P 1983 09 29/7

EXAMPLE 1

With respect to pigs, a presently preferred embodiment of the feeding method of the invention is performed according to the following parameters:

The temperature in the pigsty is varied according to the age/weight of the pigs, starting with a temperature in the range of 26-30°C and decreasing to a temperature in the range of 16-20°C according to the schedule stated below.

| Weight, kg | 6 | 9 | 12 | 15 | 18 | 21 | 24 | 27 | 30 | 33 | 36 |
|---|---|---|---|---|---|---|---|---|---|---|---|
| Temp.°C | 30 | 29 | 28 | 27 | 26 | 25 | 24 | 23 | 22 | 21 | 20 |

The number of feedings is also adjusted according to the age/weight of the animals in such a way that the number decreases with increasing weight of the animal, as outlined below.

| Weight, kg | 6 | 8 | 10 | 12 | 14 | 16 | 18 | 20 |
|---|---|---|---|---|---|---|---|---|
| No. of feedings | 18 | 17 | 16 | 15 | 14 | 13 | 12 | 11 |

| Weight, kg | 22 | 24 | 26 | 28 | 34-40 |
|---|---|---|---|---|---|
| No. of feedings | 10 | 9 | 8 | 7 | 6 |

In the weight interval from 6 to 7 kg, only protein soup is administered to the pigs. Then barley gritting is added in increasing quantities. The amount of feed to be administered to each animal is calculated on the basis of the predetermined optimal average energy intake expressed as $y = 0.06x - 0.21$, where x indicates the weight of the pigs and y indicates the number of feed units per day. During the period of restrictive feeding, the pigs are then administered an amount of feed amounting to 50-80% of the optimal average, preferably 70% of the optimal average, for 1-5 days, preferably 3 days, after which the amount of feed administered is increased to 120-150%, especially 130%, of the optimal average for 1-5 days, particularly 2 days. As an additional parameter, a diurnal rhythm may be incorporated in which a major portion of each day's feed ration, particularly about 50%, is administered early in the day such as between 6 a.m. and 12 a.m, the remainder of the feed being administered at regular intervals throughout the rest of the day.

The feed is administered on a mouthful-wise basis with a specified interval between each mouthful. The size of each mouthful varies according to the age/weight of the animals as outlined below.

| Weight, kg | 6 | 9 | 12 | 15 | 18 | 21-40 |
|---|---|---|---|---|---|---|
| Size of mouthful, g | 2 | 3 | 4 | 5 | 6 | 7 |

The interval between each mouthful is initially about 7-10 seconds, but after the passage of one third to one half of each feeding, the interval is gradually increased to about 15-20 seconds.

When a weight of 7 kg has been reached, the feed may be dosed as a homogeneous mixture of protein soup, gritting and water, but preferably the feed is dosed in such a way that pure protein soup is administered at the beginning of each feeding period until half of the prescribed amount of soup has been administered after which the amount of gritting and water is gradually increased.

The sensory stimulation of the pigs before and during feeding is established by, e.g., adjusting an acoustic generator located at the feeding apparatus to start emitting a "summoning grunt sequence" about two minutes before administration of the first mouthful, a grunt being emitted every 2.5 seconds. About 25 seconds before the first mouthful is administered, the grunting rate is accelerated so that the grunt frequency is increased to about 1.6 grunts per second. The sound emission is synchronized with the administration of the following mouthful in such a way that the grunting sound is stopped exactly at the moment the mouthful is administered. Then, there is a pause of a few seconds in the grunting. About 5 seconds before the next mouthful, a grunting sequence corresponding to the last 5 seconds of grunting prior to the first mouthful is emitted. Similarly, emission of light from a source located at each feeding place is synchronized with the supply of each mouthful in such a way that the intensity of the

0107111

light is gradually increased towards the moment when a mouthful is supplied, the intensity being greatest immediately prior to the supply of the mouthful.

EXAMPLE 2

A group of 10 pigs of an average weight of 41.2 kg were fed restrictively for one week with a conventional feed mix comprising barley gritting, soy protein, and minerals, etc., using a Biomat™ apparatus of the type described in British Patent No. 2,056,837.

During the restricive feeding period of one week, the amount of feed administered was 11% below the standard. During this period, the feed conversion dropped slightly (the ratio between consumed feed units and kg of gain increased from 1.8 to 1.9). The daily gain was reduced from 744 g to 657 on the average.

Then, overfeeding was performed for one week: The amount of feed administered was 21% above the standard. This overfeeding resulted in an increase of the feed conversion (the ratio feed units/kg gain dropped from 1.9 to 1.8), and the daily gain increased dramatically from 657 g to 1029 g on the average.

0107111

CLAIMS

1. A method of controlled feeding of animals comprising feeding each animal restrictively for a predetermined first period lasting at least one day, and preferably several days, so as to bring about increased cell division in the animal, which first period is followed by overfeeding the animal for another predetermined period, also lasting at least one day, so as to cause growth of the cells formed during the first period.

2. A method according to claim 1 in which the periods of restrictive feeding and overfeeding, respectively, are alternated until the animal has reached the desired weight.

3. A method according to claim 1 comprising exposing the animal to changes in external conditions and/or other factors influencing the animal combined with said alternation of the feeding periods.

4. A method according to claim 2 wherein the change in external conditions and/or other influencing factors comprises a change in light, sound and heat stimuli, the smell, taste and consistency of the feed, the habitat of the animal and/or the composition of the feed.

5. A method according to any of claims 1-4 comprising a period of restrictive feeding substantially lasting about 3-5 days followed by a period of overfeeding substantially lasting at least one day.

6. A method according to any of claims 1-5 in which feeding sessions are performed by simultaneously feeding a plurality of untethered animals at their respective feeding places in the same enclosure in which all feeding places are freely accessible to all the animals, and portions of feed are supplied to each of the feeding places during a feeding period at intervals ensuring that the amount of feed supplied to each feeding place substantially corresponds to a desired predetermined eating rate of the animals.

P&V F3104 jB int. tekst KBM/OP/KPJ/P 1983 09 29/7

7. A method according to any of claims 1-6 in which the animals are pre-pubescent animals.

8. A method according to any of the preceding claims in which the animals are pigs.

9. A method according to any of the preceding claims in which the decrease in the amount of feed administered to the animal during a period of restrictive feeding is in the range of 10-50%, such as 20-50%, especially 20-40%, such as 20-30%, calculated on the weight of the average optimal amount of feed, and the increase in the amount of feed during a period of overfeeding is in the range of 20-50%, in particular about 30%, calculated on the same basis.

10. A method of feeding a plurality of untethered animals at their respective feeding places in the same enclosure where all feeding places are freely accessible to all the animals, the method comprising supplying portions of feed to each feeding place during a feeding period at intervals ensuring that the amount of feed supplied to each feeding place substantially corresponds to a desired predetermined eating rate of the animals, and generating a signal at each feeding place over a period of time preceding the supply of each portion of feed, the signal being varied in the course of this period of time so as to condition the animal to expect the supply of each portion of feed at a given moment.

11. A method according to claim 10 in which the signal comprises a sound and/or light signal, the intensity of which is increased to a maximum when the portion of feed is supplied.

12. A method according to claim 11 in which the animals are pigs, and the signal comprises a reproduction of a sequence of sow suckling grunts.

13. A method according to claim 11 or 12 in which the acoustic signal is combined with a light signal.

14. A method according to any of claims 10-13 when performed in connection with a method according to any of claims 1-9.

15. A method according to any of the preceding claims in which the ratio between digestible protein and other constituents, in particular carbohydrates, in the animal feed is adapted to the development of the animals through at least substantially daily adjustment of the ratio.

16. A method according to claim 15 in which a mixture comprising digestible protein is fed in fluid form and a basic feed comprising carbohydrates is fed as a solid material, and the ratio between digestible protein and other constituents, in particular carbohydrates, in the total feed consisting substantially of the combination of the fluid mixture and the basic feed is adjusted to the development of the digestion system of the animals through at least substantially daily adjustment of the ratio between the fluid mixture and the basic feed.

17. A method according to any of claims 1-9 or 14 wherein the amount of protein fed to the animals during the period of restrictive feeding is reduced relative to the standard intake of protein for the stage of development in question, typically by an average reduction of 10-15%, and the amount of protein fed to the animals during the period of overfeeding is increased, typically by an average increase of 25-30%.

P&V F3104 jB int. tekst KBM/OP/KPJ/P 1983 09 29/7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y,A | US-A-3 386 418 (H. BIEHL)<br><br>* Claims 1, 2; column 4, line 71 - column 5, line 33 *<br><br>--- | 1,3,4,<br>8,9,11<br>,13,17 | A 01 K 5/00 |
| Y,A | COMMONWEALTH AGRICULTURAL BUREAUX, CAB Abstract 79452301 K. WALTER "Overfeeding and underfeeding of dairy cows for limited periods, and its effects on feeding costs" & Landbauforschung Volkenrode, Sonderheft 35, 1976 , pages 64-88<br><br>--- | 1,5 | |
| A | EP-A-0 023 760 (CHORE-TIME EQUIPMENT INC.)<br>* Claims 1, 2; page 4, line 19 - page 9, line 14; figure 1 *<br><br>--- | 1,6-8,<br>10 | TECHNICAL FIELDS SEARCHED (Int. Cl. ³)<br><br>A 01 K 5/00 |
| D,A | GB-A-2 056 837 (S. BLICHER)<br>* Page 2, line 104 - page 5, line 33; figures 3-6 *<br><br>--- | 1,6,10 | |
| D,P<br>A | WO-A-8 203 159 (S. BLICHER)<br><br><br>* Page 3, line 1 - page 10, line 14 *<br><br>----- | 1,3,4,<br>6-8,10<br>-13,15<br>,16 | |

The present search report has been drawn up for all claims

| Place of search | Date of completion of the search | Examiner |
|---|---|---|
| BERLIN | 20-12-1983 | BERGZOLL M C |